# EUROPEAN PATENT APPLICATION

(11) **EP 4 778 526 A1**
(43) Date of publication of application: **22.07.2026**
(21) Application number: 24865345.3
(22) Date of filing: 05.09.2024
(51) Int. Cl.: A61K 31/661, A61P 43/00

(54) **ANTI-AGING AGENT, ORAL COMPOSITION FOR ANTI-AGING, AND USE OF COMPOUND**

(30) Priority: 11.09.2023 JP 2023146775
(71) Applicant: Institute of Rheological Function of Food Co., Ltd., Kasuya-gun, Fukuoka 811-2501 (JP); Kyushu University, National University Corporation, Nishi-ku Fukuoka-shi Fukuoka 819-0395 (JP)
(72) Inventor: FUJINO Takehiko, Fukuoka-shi, Fukuoka 813-0043 (JP); MAWATARI Shiro, Fukuoka-shi, Fukuoka 813-0016 (JP); NIWASE Shamim, Fukuoka-shi, Fukuoka 810-0054 (JP); HONSHO Masanori, Fukuoka-shi, Fukuoka 819-0395 (JP); OKAUCHI Tatsuo, Kitakyushu-shi, Fukuoka 804-8550 (JP)
(74) Representative: V.O.
(86) International application number: PCT/JP2024/031798
(87) International publication number: WO 2025/057845

(57) **Abstract**

An anti-aging agent includes a compound of Formula (I), a racemate of the compound, or a salt of the compound or the racemate, as an effective component, 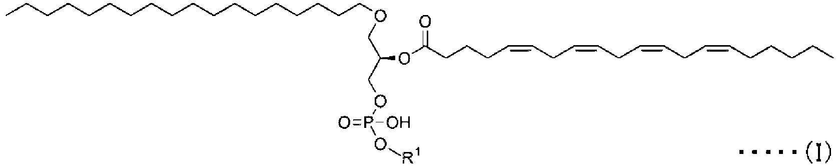 in which each carbon atom of a glycerol backbone optionally has one or more substituents, and R¹ is 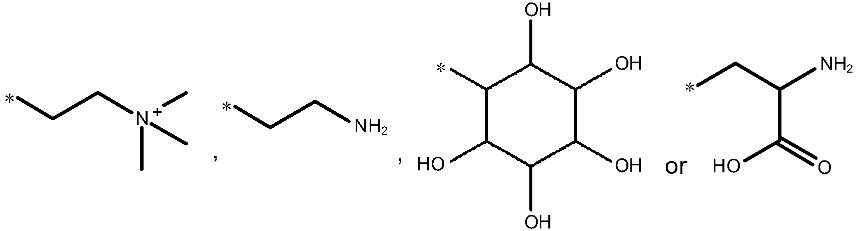 optionally having one or more substituents, in which * is an oxygen atom bound to R₁.

## Description

### Technical Field

The present disclosure relates to an anti-aging agent, an oral composition for anti-aging, and use of a compound.

### Background Art

Nicotinamide adenine dinucleotide (NAD⁺) is a universal and essential coenzyme involved in cellular redox reactions. The major biosynthetic pathway of NAD⁺ in mammals is a salvage pathway controlled by nicotinamide phosphoribosyltransferase (NAMPT) that is a rate-limiting enzyme. In this biosynthetic pathway, NAMPT converts nicotinamide into nicotinamide mononucleotide (NMN). The generated NMN is converted into NAD⁺ by nicotinamide mononucleotide adenylyltransferase (NMNAT1).

NAD⁺ functions as a coenzyme of a group of enzymes called sirtuins. Sirtuins are proteins that have functions of suppressing aging, and in humans, seven types of sirtuins (SIRT1 to SIRT7) are known. In particular, activation of SIRT1 provides various effects of suppressing aging, such as cellular rejuvenation and enhancement of metabolism.

Non Patent Literature 1 shows that caloric restriction in monkeys exhibits anti-aging effects. Non Patent Literature 2 shows that anti-aging effects by caloric restriction in mice are SIRT1-dependent. Non Patent Literature 3 shows that 25% caloric restriction for six months in humans induces expression of SIRT1. Based on such findings, anti-aging effects in humans by activation of NAMPT and SIRT1 are expected.

NAMPT and SIRT1 are known to decrease in expression with aging (Non Patent Literature 4 and Non Patent Literature 5). Patent Literature 1 discloses 2-palmitoylmonoglycerol as a substance that increases expression of NAMPT and SIRT1.

### Citation List

### Patent Literature

Patent Literature 1: Unexamined Japanese Patent Application Publication No. 2022-159178

### Non Patent Literature

Non Patent Literature 1: Ricki J. Colman, and 10 others, "Caloric restriction delays disease onset and mortality in rhesus monkeys", Science, 2009, 325(5937), 201-204
Non Patent Literature 2: Danica Chen, and three others, "Increase in Activity During Calorie Restriction Requires Sirt1", Science, 2005, 310(5754), 1641
Non Patent Literature 3: Anthony E. Civitarese, and seven others, "Calorie Restriction Increases Muscle Mitochondrial Biogenesis in Healthy Humans", PLoS Medicine, 2007, 4(3), e76
Non Patent Literature 4: Jun Yoshino, and three others, "Nicotinamide Mononucleotide, a Key NAD+ Intermediate, Treats the Pathophysiology of Diet- and Age-Induced Diabetes in Mice", Cell Metabolism, 2011, 14, 528-536
Non Patent Literature 5: Cui Chen, and three others, "SIRT1 and aging related signaling pathways", Mech Ageing Dev, 2020, 187, 111215

### Summary of Invention

### Technical Problem

Restricting caloric intake requires a long period of time to achieve desired effects, and thus may place a burden on a practitioner. To suppress various physiological declines associated with aging, there is a demand for a technique capable of safely and conveniently increasing the expression of NAMPT and SIRT1 even in elderly persons.

In view of the above circumstances, an objective of the present disclosure is to provide an anti-aging agent, an oral composition for anti-aging, and use of a compound, that are capable of increasing expression of NAMPT and SIRT1.

### Solution to Problem

An anti-aging agent according to a first aspect of the present disclosure comprises a compound of Formula (I), a racemate of the compound, or a salt of the compound or the racemate, as an effective component, 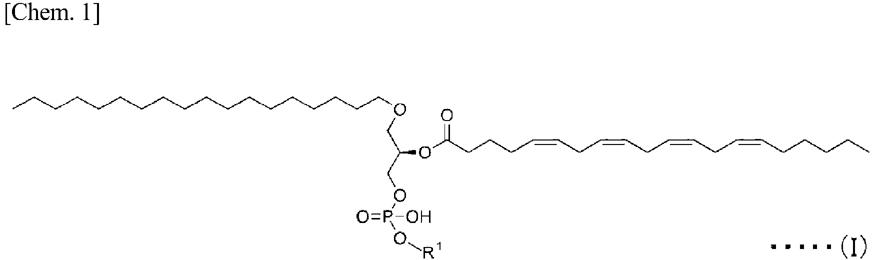 in which each carbon atom of a glycerol backbone optionally has one or more substituents, and R¹ is 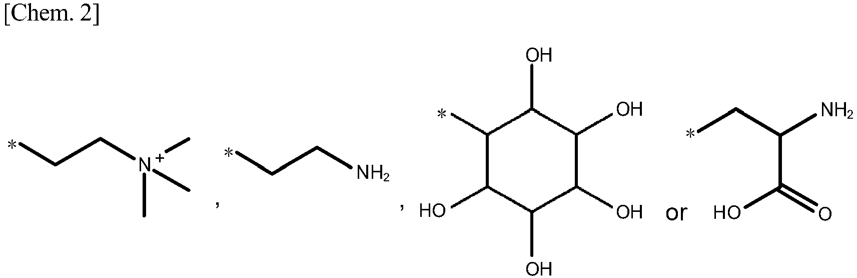 optionally having one or more substituents, in which * is an oxygen atom bound to R¹.

An oral composition for anti-aging according to a second aspect of the present disclosure comprises a compound of Formula (I), a racemate of the compound, or a salt of the compound or the racemate, as an effective component, 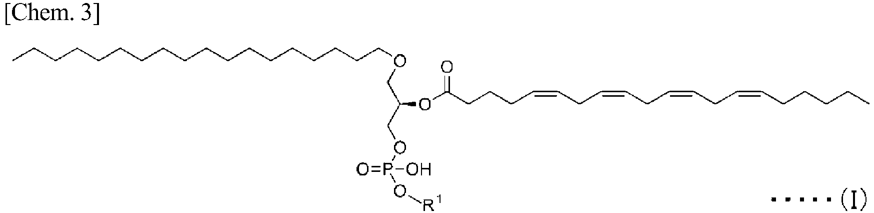 in which each carbon atom of a glycerol backbone optionally has one or more substituents, and R¹ is 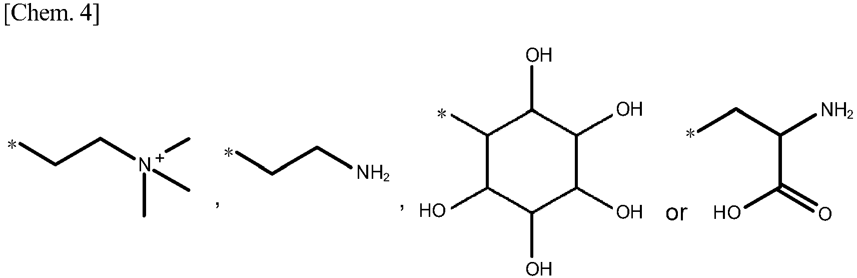 optionally having one or more substituents, in which * is an oxygen atom bound to R¹.

Use according to a third aspect of the present disclosure is use of a compound of Formula (I), a racemate of the compound, or a salt of the compound or the racemate, for producing an anti-aging agent or an oral composition for anti-aging, 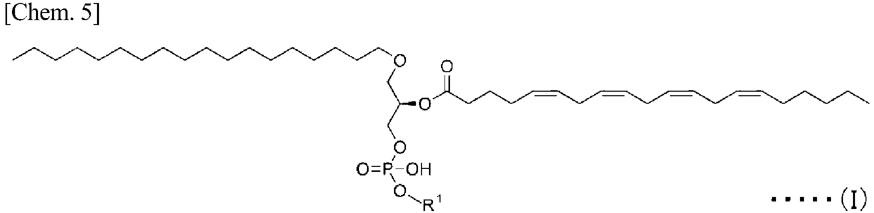 in which each carbon atom of a glycerol backbone optionally has one or more substituents, and R¹ is 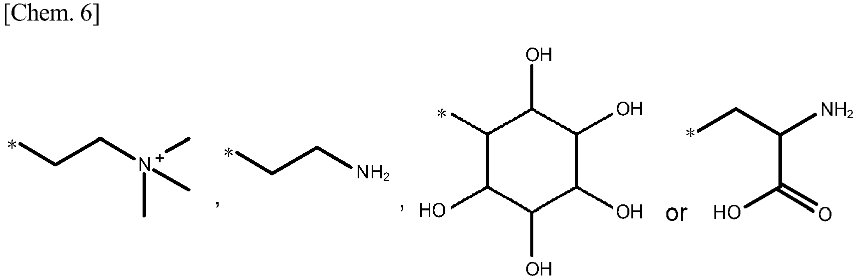 optionally having one or more substituents, in which * is an oxygen atom bound to R¹.

### Advantageous Effects of Invention

According to the present disclosure, expression of NAMPT and SIRT1 can be increased.

### Brief Description of Drawings

FIG. 1 is a diagram showing relative expression level of NAMPT in Test Example 1;
FIG. 2 is a diagram showing relative expression level of SIRT1 in Test Example 1;
FIG. 3 is a diagram showing relative expression level of NRF1 in Test Example 1;
FIG. 4 is a diagram showing relative expression level of UQCRC1 in Test Example 1;
FIG. 5 is a diagram showing relative expression level of ATP5A1 in Test Example 1;
FIG. 6 is a diagram showing relative expression level ofNAMPT in Test Example 2; and
FIG. 7 is a diagram showing relative expression level of SIRT1 in Test Example 2.

### Description of Embodiments

Embodiments according to the present disclosure are described below with reference to the drawings. In this regard, the present disclosure is not limited by the below-described embodiments or the drawings. Note that, in the below-described embodiments, the expression, "have", "include", or "contain" encompasses the meaning of "composed of' or "be constituted by".

An anti-aging agent according to an embodiment includes: a compound represented by Formula (I), a racemate of the compound, or a salt of the compound or the racemate (hereinafter also referred to as "compound or the like"), as an effective component. Each carbon atom of the glycerol backbone in Formula (I) may have one or more substituents. Examples of the substituents of the carbon atom of the glycerol backbone include hydroxy, halogen, aryl, C1-C4 alkyl, and C1-C4 alkoxy.

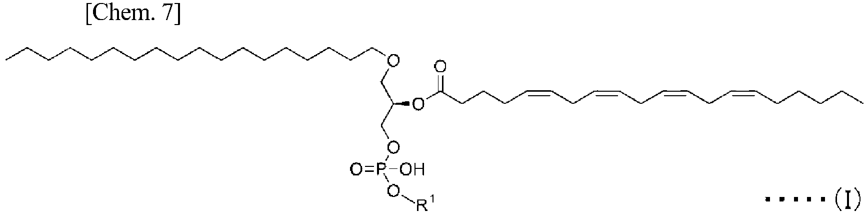

In Formula (I), R¹ is 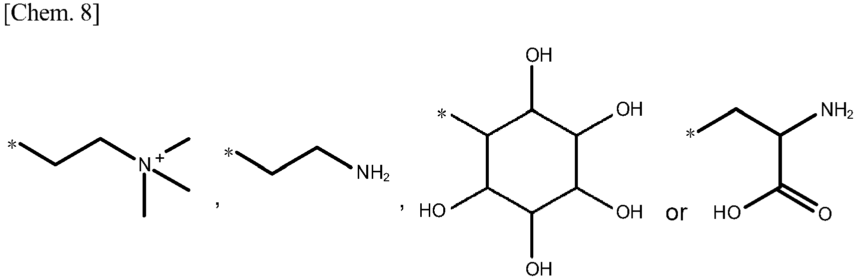 optionally having one or more substituents, in which * is an oxygen atom bound to R¹. Examples of the substituents of R¹ include hydroxy, halogen, aryl, C1-C4 alkyl, and C1-C4 alkoxy.

The compound according to the present embodiment is preferably represented by Formula (II) below.

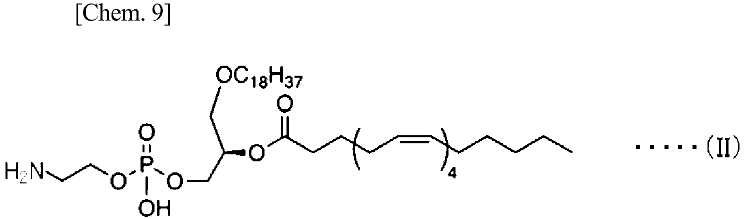

The salt of the compound is not particularly limited, but is preferably a pharmaceutically acceptable salt. The salt of the compound may be either an acidic salt or a basic salt. Examples of the salts include alkali metal salts such as lithium salt, sodium salt, and potassium salt; alkaline earth metal salts such as magnesium salt and calcium salt; inorganic acid salts such as hydrochloride, hydrobromide, sulfate, nitrate, oxalate, and phosphate; and organic acid salts such as acetate, propionate, hexanoate, cyclopentane propionate, glycolate, pyruvate, lactate, malonate, succinate, malate, fumarate, tartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, camphorsulfonate, glucoheptanoate, 3-phenylpropionate, trimethylacetate, tertiary butyl acetate, lauryl sulfate, gluconate, glutamate, hydroxynaphthoate, salicylate, stearate, trifluoroacetate (TFA), maleate, and muconate.

The compound or the like according to the present embodiment can be synthesized by a known method. For example, in cases where a compound containing ethanolamine as -O-R¹ is to be synthesized, first, arachidonic acid is reacted with tert-butyl(2-((tert-butoxy((R)-2-hydroxy-3-(octadecyloxy)propoxy)phosphoryl)oxy)ethyl)carbamate using a condensing agent. The resulting product is deprotected by allowing a strong acid such as trifluoroacetic acid to act on the product, to obtain a compound according to the present embodiment. The condensing agent is not limited, and may be, for example, 1-(3-dimethylaminopropyl)-3-ethylcarbodiimide (EDC) hydrochloride, dicyclohexylcarbodiimide (DCC), HATU, HBTU, TATU, TBTU, and diphenylphosphoryl azide (DPPA). For promoting the dehydration condensation reaction by the condensing agent, a nucleophile such as dimethylaminopyridine (DMAP) may be used.

The content of plasmalogen in the anti-aging agent according to the present embodiment is appropriately adjusted. For example, the anti-aging agent includes the compound or the like at 0.000001 to 99.9% by mass, 0.00001 to 99.8% by mass, 0.0001 to 99.7% by mass, 0.001 to 99.6% by mass, 0.01 to 99.5% by mass, 0.1 to 99% by mass, 0.5 to 60% by mass, 1 to 50% by mass, or 1 to 2% by mass as an effective component.

Subjects to which the anti-aging agent according to the present embodiment is administered are cells, biological tissue, organisms, animals, or the like. The anti-aging agent as a pharmaceutical is administered to humans or animals. Preferred examples of the animals include mammals, more specifically, dogs, cats, cows, pigs, horses, sheep, and deer.

The administration route of the anti-aging agent according to the present embodiment for humans or the like is not limited. The anti-aging agent is preferably used as an external preparation, an injection solution, or an orally administered agent. The anti-aging agent may include, for example, the compound or the like and a pharmaceutically acceptable carrier. The term "pharmaceutically acceptable carrier" refers to any of various types of organic carrier substances and inorganic carrier substances that are used as materials for pharmaceutical preparations. The pharmaceutically acceptable carriers are, for example, excipients, binders, disintegrants, lubricants, stabilizers, and flavoring and deodorizing agents in solid preparations, or solvents, solubilizing agents, suspending agents, tonicity agents, buffering agents, and flavoring and deodorizing agents in liquid preparations. An additive such as antiseptic agents, antioxidants, coloring agents, or sweeteners may also be optionally blended.

Examples of the excipients include lactose, white sugar, D-mannitol, D-sorbitol, starch, α-starch, dextrin, crystalline cellulose, low-substituted hydroxypropyl cellulose, sodium carboxymethylcellulose, gum arabic, pullulan, light anhydrous silicic acid, synthetic aluminum silicate, magnesium aluminometasilicate, xylitol, sorbitol, and erythritol.

Examples of the binders include, in addition to the excipients above, α-starch, sucrose, gelatin, gum arabic, methyl cellulose, carboxymethyl cellulose, sodium carboxymethylcellulose, crystalline cellulose, white sugar, D-mannitol, trehalose, dextrin, pullulan, hydroxypropyl cellulose, hydroxypropylmethylcellulose, and polyvinylpyrrolidone.

Examples of the disintegrants include, in addition to the excipients above, lactose, white sugar, starch, carboxymethyl cellulose, carboxymethyl cellulose calcium, croscarmellose sodium, carboxymethyl starch sodium, low-substituted hydroxypropyl cellulose, light anhydrous silicic acid, and calcium carbonate.

Examples of the lubricants include magnesium stearate, calcium stearate, talc, colloidal silica, and polyethylene glycol.

Examples of the stabilizers include paraoxybenzoic acid esters such as methylparaben and propylparaben; alcohols such as chlorobutanol, benzyl alcohol, and phenylethyl alcohol; benzalkonium chloride; acetic anhydride; and sorbic acid. Examples of the flavoring and deodorizing agents include sweeteners, acidulants, and flavors.

Examples of the solvents include water for injection, physiological saline, Ringer solution, alcohol, propylene glycol, polyethylene glycol, sesame oil, corn oil, olive oil, and cottonseed oil. Examples of the solubilizing agents include polyethylene glycol, propylene glycol, D-mannitol, trehalose, benzyl benzoate, ethanol, tris(hydroxymethyl)aminomethane, cholesterol, triethanolamine, sodium carbonate, sodium citrate, sodium salicylate, and sodium acetate.

Examples of the suspending agents include surfactants such as stearyltriethanolamine, sodium lauryl sulfate, lauryl aminopropionic acid, lecithin, benzalkonium chloride, benzethonium chloride, and glycerol monostearate; hydrophilic polymers such as polyvinyl alcohol, polyvinylpyrrolidone, sodium carboxymethylcellulose, methyl cellulose, hydroxymethylcellulose, hydroxyethylcellulose, and hydroxypropyl cellulose; polysorbates; and polyoxyethylene hydrogenated castor oils.

Examples of the tonicity agents include sodium chloride, glycerin, D-mannitol, D-sorbitol, glucose, xylitol, and fructose. Examples of the buffering agents include buffer solutions such as phosphate, acetate, carbonate, and citrate buffers.

Examples of the antiseptic agents include paraoxybenzoic acid esters, chlorobutanol, benzyl alcohol, phenethyl alcohol, dehydroacetic acid, and sorbic acid. Examples of the antioxidants include sulfite and ascorbic acid. Examples of the coloring agents include water-soluble tar dyes, lake pigments, and natural colorants. Examples of the sweeteners include sodium saccharin, dipotassium glycyrrhizinate, aspartame, and stevia.

The anti-aging agent according to the present embodiment is produced by a known method, and provided in the form of liquids, creams, ointments, pills, granules, fine granules, powders, tablets, capsules, or the like.

The dose of the anti-aging agent according to the present embodiment is determined as appropriate depending on, for example, the age, body weight, and symptom of a human or animal to which the anti-aging agent is administered. The anti-aging agent is administered in such an amount that an effective amount of the compound or the like is contained therein. The effective amount is an amount of the compound or the like required for obtaining a desired result, and is an amount required for delaying, inhibiting, preventing, or reversing the progression of, or for curing of, the condition to be treated or handled.

The dose of the anti-aging agent according to the present embodiment is, for example, 0.000001 µg or more per day for an adult, preferably 1 µg or more or 500 µg or more, and more preferably 1000 µg or more. The upper limit is, for example, 20000 µg per day, preferably 10000 µg. The anti-aging agent can be administered once or multiple times per day. Further, the anti-aging agent may be administered in any of various types of dosing frequencies, such as, for example, every other day, once a week, every other week, and once a month. If necessary, a dose other than the above-described range can also be used.

The compound or the like that is an effective component in the anti-aging agent according to the present embodiment increases expression of NAMPT and SIRT1 as shown in the Examples below. As a result, anti-aging effects are obtained.

The compound or the like according to the present embodiment may be used as a reagent for the purpose of enhancing expression of NAMPT, SIRT1, NRF1, UQCRC1, and ATP5A1 in in vitro and in vivo experiments.

In another aspect of the present embodiment, an oral composition for anti-aging is provided. Specific examples of such an oral composition include supplements, food and beverage compositions, functional foods, and food additives.

The forms of the supplements are not limited, and may be any of tablets, powders, granules, capsules, sugar-coated tablets, film formulations, troches, chewable formulations, solutions, emulsions, suspensions, and the like. The supplements may include any components that are normally used for supplements.

"Functional food" means a food or beverage consumed for the purpose of maintaining health, and examples of the functional food include: foods for specified health uses, foods with function claims, and foods with nutrient function claims that are foods with health claims; health foods; dietary supplements; and the like. The functional foods are preferably foods for specified health uses or foods with nutrient function claims that are foods with health claims. In cases of commercialization as a functional food, the oral composition for anti-aging may include various additives for use in foods, such as colorants, preservatives, thickening stabilizers, antioxidants, bleaching agents, antibacterial and antifungal agents, acidulants, sweeteners, seasonings, emulsifiers, fortifiers, agents for production, flavors, and the like.

The functional food may be either a food or beverage, and is not limited as long as the functional food can be orally taken. Examples of the forms of the functional food include beverages, confectionery, processed cereal products, kneaded products, dairy products, and seasonings. Examples of the beverages include nutritional drinks, soft drinks, black tea, and green tea. Examples of the confectionery include candies, cookies, tablets, chewing gums, and jellies. Examples of the processed cereal products include noodle, bread, cooked rice, and biscuits. Examples of the kneaded products include sausage, ham, and kamaboko that is a boiled fish paste. Examples of the dairy products include butter and yogurt.

The oral composition for anti-aging may be added to foods as food additives. In such a case, examples of the food additives may include pastes, gel-like agents, powders, liquids, suspensions, emulsions, granules, and the like, from the viewpoint of easily adding the food additive to foods.

The oral composition for anti-aging according to the present embodiment may contain water, vitamins, minerals, organic acids, organic bases, fruit juices, flavors, functional components, food additives, and the like, as long as the oral composition for anti-aging maintains anti-aging effects. The oral composition for anti-aging may be produced by a known method in which components other than the aforementioned compound, or effective components, are added, as necessary.

The oral composition for anti-aging may be divided into one or more containers such that the daily intake is in accordance with the above-described amount of intake. In such a case, the oral composition for anti-aging is preferably contained in each container in an amount corresponding to the daily intake.

From the viewpoint of the fact that the oral composition for anti-aging contains the aforementioned compound or the like and is used for anti-aging, the oral composition for anti-aging is provided in the forms of products that can be distinguished from other products. For example, at least one of the product packaging, instructions, and advertisements for the oral composition for anti-aging indicates that the oral composition for anti-aging has anti-aging effects.

When the oral composition for anti-aging according to the present embodiment is provided as a food and beverage composition, the oral composition for anti-aging may be offered or sold as a food or beverage with an indication of the application for preventing aging, reducing aging-related risks, and the like, in addition to anti-aging effects. The application includes health purposes. The act of "indication" includes all acts that inform consumers of the aforementioned application. Any expression that evokes or suggests the aforementioned application is considered an act of "indication", regardless of the purpose, content, object, medium, and the like of the indication.

The "indication" is preferably made in a manner that allows the consumers to directly recognize the aforementioned application. Specifically, this includes acts such as an act of transferring, delivering, exhibiting for transfer or delivery, or importing food and beverage products or product packaging with the aforementioned application described; an act of exhibiting or distributing advertisements, price lists, or transaction documents relating to the products with the aforementioned application described, or providing information of the content with the aforementioned application described via electromagnetic means such as the Internet.

The "indication" may include indications as health foods, functional foods, enteral nutritional foods, foods for special uses, foods with health claims, foods for specified health uses, foods with nutrient function claims, foods with function claims, quasi-drugs, and the like. Among these, particularly, indications approved under systems for foods for specified health uses, foods with nutrient function claims, or foods with function claims, or similar systems are included. Specifically, examples of the indications include an indication as foods for specified health uses, an indication as conditional foods for specified health uses, an indication of effects on body structure or function, an indication for disease risk reduction, and an indication of functionality based on scientific evidence. More specifically, typical examples thereof include an indication as foods for specified health uses, in particular, an indication of health purposes, and similar indications.

In still another aspect of the present embodiment, use of the aforementioned compound or the like for producing the anti-aging agent or the oral composition for anti-aging is provided. In yet another aspect of the present embodiment, a method of anti-aging including a step of administering the aforementioned compound or the like to a subject is provided. In a further aspect of the present embodiment, the aforementioned compound or the like for the use in anti-aging is provided.

The present disclosure is more specifically described with reference to the following Examples, but the present disclosure is not limited to the Examples.

### Examples

### Example 1: Synthesis of Compound KIT13

KIT13 was synthesized as a compound according to Formula (I) as follows.

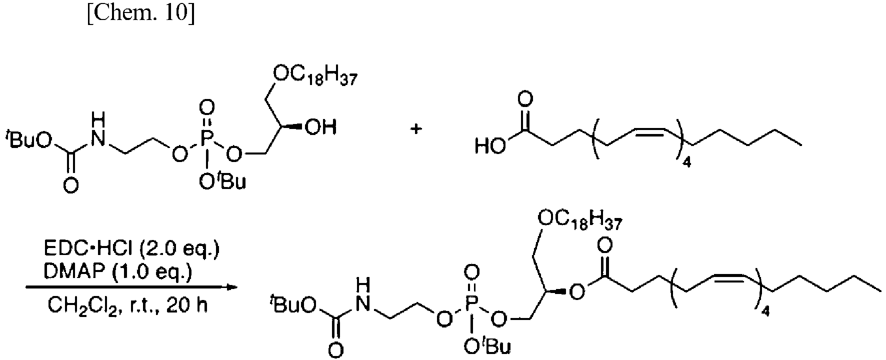

To a solution of tert-butyl(2-((tert-butoxy((R)-2-hydroxy-3-(octadecyloxy)propoxy)phosphoryl)oxy)ethyl)carbamate (187 mg, 0.30 mmol) in dichloromethane (3 mL), EDC hydrochloride (115 mg, 0.60 mmol), DMAP (37 mg, 0.30 mmol), and arachidonic acid (100 mg, 0.33 mmol) were added at room temperature, and the resulting mixture was stirred for 20 hours. The reaction solution was diluted with ethyl acetate, and the resulting dilution was washed with water. The ethyl acetate layer was dried over anhydrous sodium sulfate, and then concentrated under reduced pressure. The residue was purified by column chromatography, to obtain a desired product, (2R)-1-((tert-butoxy(2-((tert-butoxycarbonyl)amino)ethoxy)phosphoryl)oxy)-3-(octadecyloxy)propan-2-yl(5Z,8Z,11Z,14Z)-icosa-5,8,11,14-tetraenoate, as a colorless liquid (260 mg, 95%).

¹H NMR (500 MHz, CDCl₃) δ 5.43-5.31 (m, 8H), 5.16 (quin, J = 4.9 Hz, 1H), 5.16 (br, 1H), 4.21 (m, 1H), 4.14-4.09 (m, 1H), 4.07-4.02 (m, 2H), 3.55 (d, J = 5.3 Hz, 2H), 3.47-3.38 (m, 5.3 Hz), 2.85-2.79 (m, 6H), 2.35 (t, J = 7.6 Hz), 2.12 (q, J = 7.0 Hz, 2H), 2.06 (q, J = 7.1 Hz, 2H), 1.74-1.67 (m, 2H), 1.56-1.54 (m, 2H), 1.502 (s, 9H, one diastereomer), 1.496 (s, 9H, the other diastereomer), 1.39-1.25 (m, 38H), 0.90-0.87 (m, 6H).

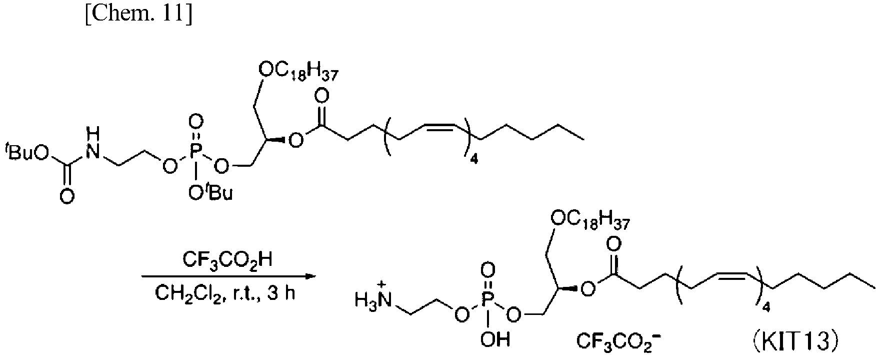

To a solution of (2R)-1-((tert-butoxy(2-((tert-butoxycarbonyl)amino)ethoxy)phosphoryl)oxy)-3-(octadecyloxy)propan-2-yl(5Z,8Z,11Z,14Z)-icosa-5,8,11,14-tetraenoate (260 mg, 0.29 mmol) in dichloromethane (2 mL), 2 mL of trifluoroacetic acid was added at room temperature, and the resulting mixture was stirred for 3 hours. The reaction mixture was concentrated under reduced pressure, to obtain a desired product, 2-(hydroxy((R)-2-(((5Z,8Z,11Z,14Z)-icosa-5,8,11,14-tetraenoyl)oxy)-3-(octadedecyloxy)propoxy)phosphoryl)oxy)ethane-1-ammonium 2,2,2-trifluoroacetate (KIT13), as a colorless liquid (249 mg, quant.).

¹H NMR (500 MHz, CDCl₃) δ 11.31 (br, 1H), 5.41-5.31 (m, 8H), 5.16-5.15 (m, 1H), 4.17 (br, 2H), 4.07-4.00 (m, 2H), 3.54 (d, J = 4.9 Hz, 2H), 3.45-3.39 (m, 2H), 3.27 (br), 2.84-2.78 (m, 6H), 2.35 (t, J = 7.4 Hz, 2H), 2.10 (q. J = 7.1 Hz, 2H), 2.05 (q. J = 7.2 Hz, 2H), 1.69-1.66 (m, 2H), 1.54-1.51 (m, 2H), 1.39-1.25 (m, 38H), 0.90-0.86 (m, 6H).

### Test Example 1: Study of Effects of Compound KIT13 on Expression Levels of NAMPT and SIRT1.

Human hair outer root sheath cells (HHORSCs; manufactured by ScienCell Research Laboratories, #2420) were cultured for 7 hours in a culture medium containing 5 µg/ml of KIT13. As a culture medium for HHORSCs, a mesenchymal stem cell medium (manufactured by ScienCell Research Laboratories, #7501) was used. As a negative control, HHORSCs were cultured in a culture medium not containing KIT13.

RNA was extracted from cells using TRIzol (manufactured by Thermo Fisher Scientific). Subsequently, a first strand cDNA was prepared using PrimeScript RT Master Mix (manufactured by Takara), and mRNA expression of target genes and endogenous control genes was detected by real-time PCR using TB Green Premix Ex Taq II (manufactured by Takara) and T100 (trademark) Thermal Cycler (manufactured by BIO-RAD). The target genes were NAMPT, SIRT1, and NRF1, UQCRC1, and ATP5A1 whose expression is induced by activation of SIRT1 (Yuanyuan Huang, and ten others, "Resveratrol-induced Sirt1 phosphorylation by LKB1 mediates mitochondrial metabolism", J. Biol. Chem., 2021, 297(2), 100929). As the endogenous control gene, 18S ribosomal RNA (18S rRNA) was used, and measurement values of each gene were normalized by measurement values of 18S rRNA. The base sequences of primers used in the real-time PCR are shown in Table 1.

**Table 1**

| | FORWARD PRIMER | | REVERSE PRIMER | |
|---|---|---|---|---|
| NAMPT | gccagcagggaattttgtta | (SEQ ID NO: 1) | tgtcaccttgccattcttga | (SEQ ID NO: 2) |
| SIRT1 | tcagtggctggaacagtgag | (SEQ ID NO: 3) | agcgccatggaaaatgtaac | (SEQ ID NO: 4) |
| NRF1 | gtccagatccctgtgagcat | (SEQ ID NO: 5) | caatgtcaccacctccacag | (SEQ ID NO: 6) |
| UQCRC1 | tgtctcgtgcagacttgacc | (SEQ ID NO: 7) | tctgggcgaggtctaacag | (SEQ ID NO: 8) |
| ATP5A1 | attggttccaagacgcgtag | (SEQ ID NO: 9) | attggcaccaagctatccac | (SEQ ID NO: 10) |
| 18S rRNA | agtccctgccctttgtacaca | (SEQ ID NO: 11) | cgatccgagggcctcacta | (SEQ ID NO: 12) |

### Results

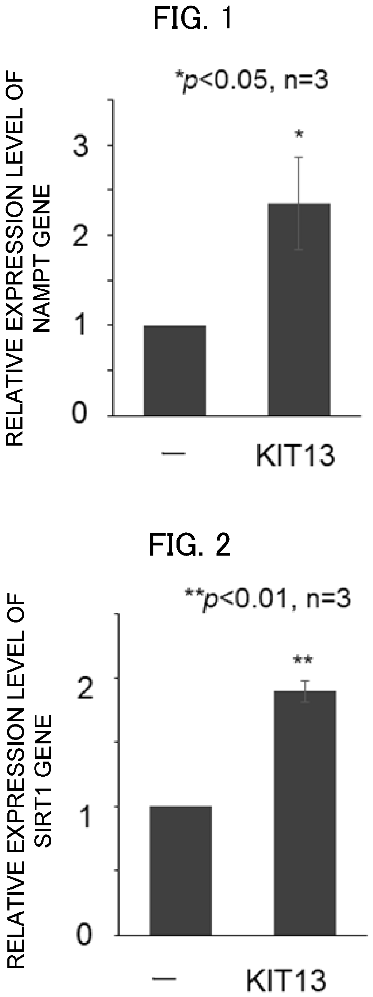

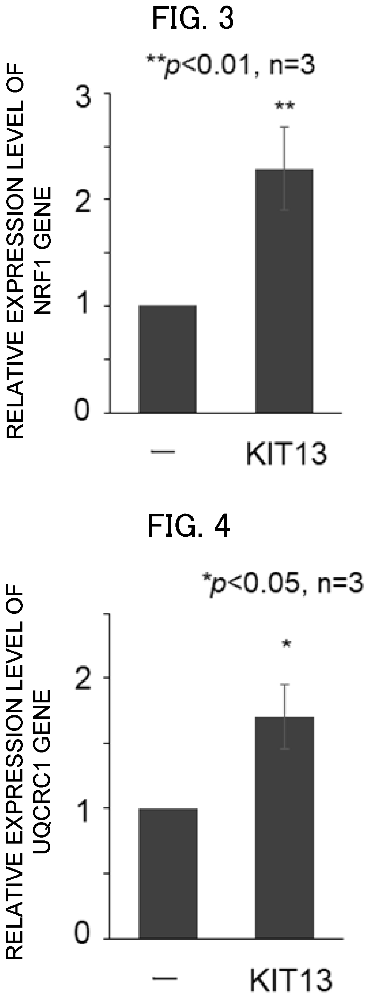

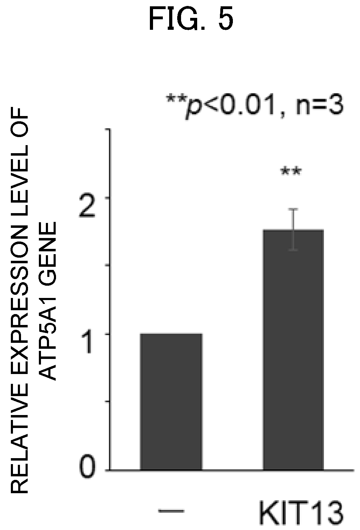

As shown in FIGS. 1 and 2, KIT13 significantly increased expression levels of NAMPT and SIRT1. Furthermore, as shown in FIGS. 3, 4, and 5, expression levels of NRF1, UQCRC1, and ATP5A1 were also increased. The increase in expression levels of NRF1, UQCRC1, and ATP5A1 is dependent on activation of SIRT1.

### Test Example 2: Study of Effects of Compound KIT13 on Expression Levels of NAMPT and SIRT1 in mice in vivo.

KIT13 was administered to four-week-old male c57BL6J mice (five mice per group, n = 5) by drinking water at a dose of 20 µg/kg for four weeks. Femoral muscles were collected from each mouse and analyzed as follows.

Femoral muscles were crushed by homogenization using 18G to 25G injection needles in Buffer A (0.25 M sucrose, 10 mM Hepes-KOH (pH 7.5), 1 mM EDTA, protease inhibitor cocktail), and RNA was extracted from the cells based on equal amounts of protein using TRIzol (manufactured by Thermo Fisher Scientific). Subsequently, a first strand cDNA was prepared using PrimeScript RT Master Mix (manufactured by Takara), and mRNA expression of target genes and endogenous control genes was detected by real-time PCR using TB Green Premix Ex Taq II (manufactured by Takara) and T100 (trademark) Thermal Cycler (manufactured by BIO-RAD). The target genes were NAMPT and SIRT1, and the endogenous control gene was β-actin. Measurement values of each gene were normalized by measurement values of β-actin. The base sequences of primers used in the real-time PCR are shown in Table 2.

**Table 2**

| | FORWARD PRIMER | | REVERSE PRIMER | |
|---|---|---|---|---|
| NAMPT | cataggggcatctgctcatt | (SEQ ID NO: 13) | gctgctggaacagaatagcc | (SEQ ID NO: 14) |
| SIRT1 | agttccagccgtctctgtgt | (SEQ ID NO: 15) | gatcctttggattcctgcaa | (SEQ ID NO: 16) |
| *β*-actin | gatctggcaccacaccttct | (SEQ ID NO: 17) | ggggtgttgaaggtctcaaa | (SEQ ID NO: 18) |

### Results

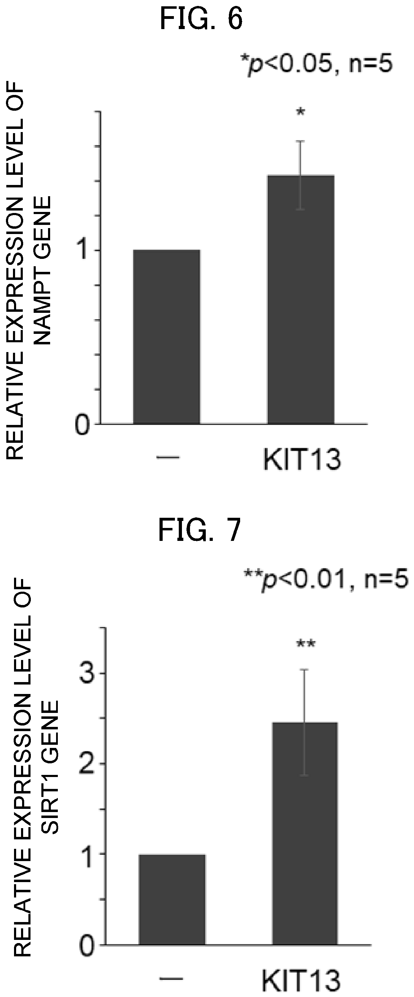

As shown in FIGS. 6 and 7, KIT13 significantly increased expression levels of NAMPT and SIRT1 in muscle.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-146775, filed on September 11, 2023, the entire disclosure of which is incorporated by reference herein.

### Industrial Applicability

The present disclosure is useful for anti-aging agents.

## Claims

1. An anti-aging agent, comprising:
a compound of Formula (I), a racemate of the compound, or a salt of the compound or the racemate, as an effective component, 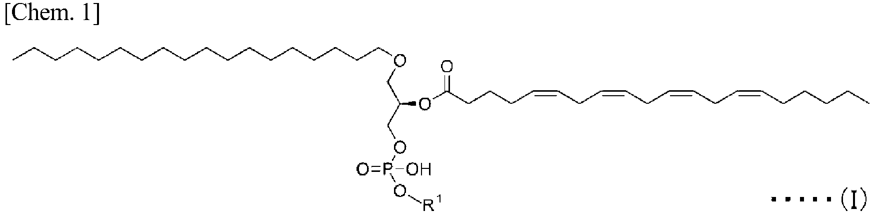 in which each carbon atom of a glycerol backbone optionally has one or more substituents, and R¹ is 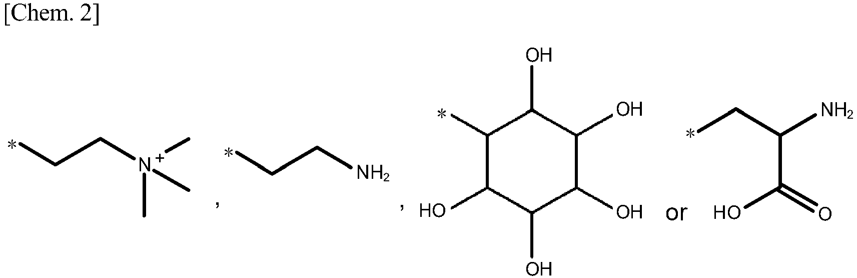 optionally having one or more substituents, in which * is an oxygen atom bound to R¹.

2. An oral composition for anti-aging, comprising:
a compound of Formula (I), a racemate of the compound, or a salt of the compound or the racemate, as an effective component, 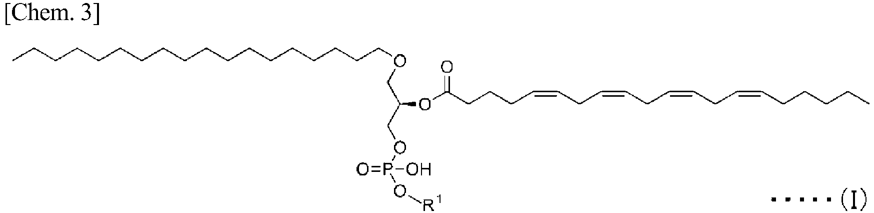 in which each carbon atom of a glycerol backbone optionally has one or more substituents, and R¹ is 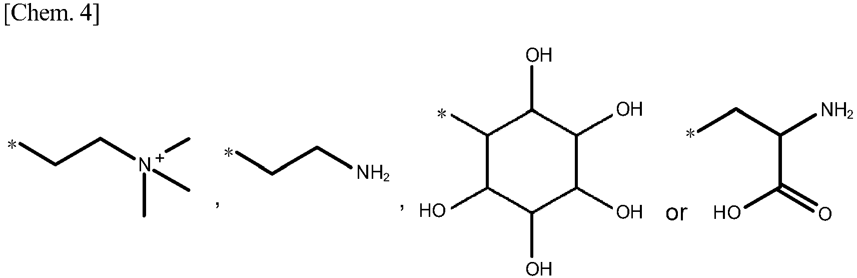 optionally having one or more substituents, in which * is an oxygen atom bound to R¹.

3. Use of a compound of Formula (I), a racemate of the compound, or a salt of the compound or the racemate, for producing an anti-aging agent or an oral composition for anti-aging, 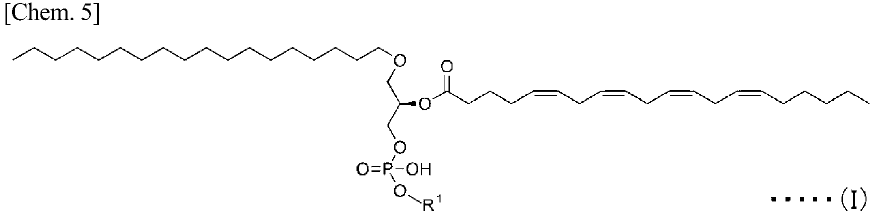 in which each carbon atom of a glycerol backbone optionally has one or more substituents, and R¹ is 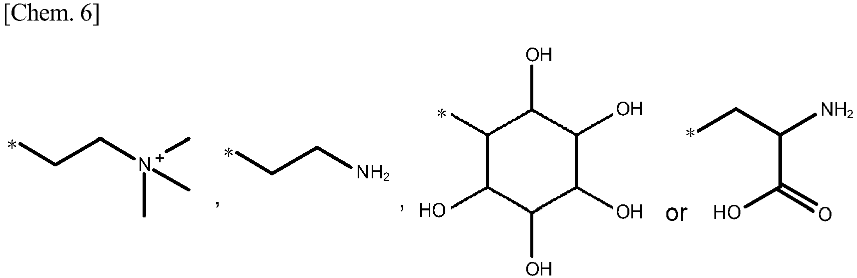 optionally having one or more substituents, in which * is an oxygen atom bound to R¹.
